# EUROPÄISCHE PATENTANMELDUNG

(11) **EP 2 551 668 A2**
(43) Veröffentlichungstag der Anmeldung: **30.01.2013**
(21) Anmeldenummer: 12178102.5
(22) Anmeldetag: 26.07.2012
(51) Int. Cl.: G01N 29/11, G01S 13/00, G01S 15/00

(54) **Vorrichtung zur Schneebeschaffenheitsmessung**

(30) Priorität: 27.07.2011 AT 10962011
(71) Anmelder: SET Software Engineering Tschürtz GmbH, 7210 Mattersburg (AT)
(72) Erfinder: Fischer, Clemens, 2345 Brunn am Gebirge (AT); Lümen, Reinhard, 2700 Wr. Neustadt (AT); Tschürtz, Johann, 7210 Mattersburg (AT)
(74) Vertreter: Schwarz & Partner

(57) **Zusammenfassung**

Die Erfindung betrifft eine Vorrichtung (1) zur Schneebeschaffenheitsmessung, umfassend zumindest eine meteorologische Messeinrichtung (9) und/oder eine Schneehöhen-Messeinrichtung (10) und/oder eine Schneewiegeeinrichtung (11) und/oder eine Schneetemperatur-Messeinrichtung (12) und/oder eine Schneedichte-Messeinrichtung (13) und/oder eine Schneefeuchte-Messeinrichtung (14) und eine Schneeschichten-Messeinrichtung (15), mit der ein stetiges Schneeschichtenprofil erfassbar ist. Die Schneeschichten-Messeinrichtung (15) weist dabei eine Impulsreflektometrie-Messeinrichtung (36) umfassend zumindest eine Strom leitende Impulsreflektometrie-Messstrecke (41) auf.

## Beschreibung

Die Erfindung betrifft eine Vorrichtung zur Schneebeschaffenheitsmessung mit den Merkmalen des Oberbegriffs von Anspruch 1.

Aus dem Stand der Technik sind unterschiedliche Vorrichtungen zur Schneebeschaffenheitsmessung bekannt, welche bislang vorwiegend bei meteorologischen Messstationen eingesetzt werden. Mit derartigen Vorrichtungen werden beispielsweise die Schneehöhe, das Schneegewicht mittels einer Schneewaage und die Temperatur der Schneeoberfläche mittels eines Infrarotsensors sowie der Wassergehalt der Schneedecke gemessen. Weiters ist bekannt, für eine Schneedichte-Messeinrichtung beispielsweise eine Laserpuls-Messeinrichtung (LIDAR, abgekürzt für Light Detection And Ranging) zu verwenden.

Nachteilig an den aus dem Stand der Technik bekannten Vorrichtungen zur Schneebeschaffenheitsmessung ist, dass eine möglichst stetige Erfassung eines Schneefestigkeitsprofils bzw. einer Schneedeckenschichtung nicht vorgesehen ist. Bei den derzeit bekannten Schneeschichten-Messvorrichtungen wird die Fließgeschwindigkeit der Schneedecke nicht erfasst, was einen weiteren Nachteil des Stands der Technik darstellt. Die Fließgeschwindigkeit der Schneedecke ist einer der wesentlichen Parameter für eine Lawinengefahrenanalyse.

Es ist somit die Aufgabe der vorliegenden Erfindung eine Vorrichtung zur Schneebeschaffenheitsmessung bereitzustellen, die die geschilderten Nachteile des Stands der Technik vermeidet.

Diese Aufgabe wird bei einer Vorrichtung zur Schneebeschaffenheitsmessung gemäß dem Oberbegriff des Anspruches 1 mit den Merkmalen des kennzeichnenden Teiles des Anspruchs 1 gelöst.

Vorteilhafte Ausgestaltungen und Fortbildungen der Erfindung sind in den Unteransprüchen und der Beschreibung dargelegt.

Bei einer erfindungsgemäßen Vorrichtung zur Schneebeschaffenheitsmessung, umfassend zumindest eine meteorologische Messeinrichtung und/oder eine Schneehöhen-Messeinrichtung und/oder eine Schneewiegeeinrichtung und/oder eine Schneetemperatur-Messeinrichtung und/oder eine Schneedichte-Messeinrichtung und/oder eine Schneefeuchte-Messeinrichtung und eine Schneeschichten-Messeinrichtung, mit der ein stetiges Schneeschichtenprofil erfassbar ist, weist die Schneeschichten-Messeinrichtung eine Impulsreflektometrie-Messeinrichtung umfassend zumindest eine Strom leitende Impulsreflektometrie-Messstrecke auf.

Eine Schneeschichten-Messeinrichtung zur Erfassung eines stetigen Schneeschichtenprofils kann nach unterschiedlichen Messprinzipien erfolgen. Wesentlich ist, dass von einer erfindungsgemäßen Vorrichtung ein Schichtenprofil einer Schneedecke jeweils stetig, also kontinuierlich durchgängig über die Höhe der Schneedecke aufgenommen wird. Somit werden sämtliche Schichtgrenzflächen innerhalb der Schneedecke erfasst.

Weiters kann im Rahmen der Erfindung mittels einer Schneeschichten-Messeinrichtung eine Schichtenentstehung einer Schneedecke erfasst werden. Dazu wird von der Schneeschichten-Messeinrichtung in gleichmäßigen Zeitintervallen das Wachstum bzw. die Veränderung des Schneedeckenaufbaus erfasst und beispielsweise von einer entsprechenden Auswerte- bzw. Erfassungseinheit ausgewertet.

Meteorologische Messdaten, die von einer oder mehreren meteorologischen Messeinrichtungen aufgezeichnet werden können, sind beispielsweise die Windgeschwindigkeit, die Windrichtung, Lufttemperatur, Luftdruck, Luftfeuchte, Globalstrahlung, Schneedeckenabstrahlung, Schneeoberflächentemperatur, die Schneehöhe sowie die Niederschlagsmenge.

Besonders zweckmäßig weist bei einer Vorrichtung gemäß der Erfindung die Schneeschichten-Mes seinrichtung zumindest einen Schallkopf umfassend einen Ultraschallsender/-empfänger auf.

Vorteilhaft ist bei einer erfindungsgemäßen Vorrichtung zumindest ein Schallkopf vorgesehen, welcher vorzugsweise in Bodennähe der Vorrichtung angeordnet ist, und welcher eine nach oben ausgerichtete Schallabstrahlrichtung aufweist.

Ein Schallkopf umfassend einen Ultraschallsender/-empfänger ist dabei in Bodennähe der Vorrichtung nach oben ausgerichtet montiert und befindet sich somit in der untersten Schneeschicht der Schneedecke. Der Schallkopf sendet Schallwellen aus, die in der untersten sowie in allen weiteren Schneeschichten bzw. an den Schichtgrenzflächen zwischen den einzelnen Schneeschichten zumindest teilweise reflektiert werden. Der Schallkopf ist so justiert, dass ein Abstrahlwinkel der ausgesendeten Schallwellen im Wesentlichen normal auf die Schneeschichtgrenzflächen bzw. auf die Oberfläche der Schneedecke trifft. Somit ist der Abstrahlwinkel der Schallwellen weiters im Wesentlichen normal zu einer Geländeoberfläche am Aufstellort der Vorrichtung bzw. normal auf eine Grundplatte, mit der die Vorrichtung auf einem Geländeboden befestigt ist.

Dabei wird das Prinzip der Teilreflexion von Schallwellen an Schichten bzw. Medien mit unterschiedlicher Dichte genutzt. Die an den Schichtgrenzflächen teilweise reflektierten Schallwellen gelangen jeweils zeitversetzt wiederum zum Ultraschallsender/-empfänger zurück. Somit wird vom Ultraschallsender/-empfänger im Wesentlichen die gesamte Schneedecke beschallt und die Anzahl und Lage sämtlicher Schneeschichten innerhalb der Schneedecke erfasst. Bei der Auswertung der aufgezeichneten Schallamplituden als Funktion der Zeit können einzelne Amplitudensignale jeweils den Schichtgrenzflächen zwischen benachbarten Schneeschichten zugeordnet werden. Somit kann die Anzahl, Lage sowie Schichtdicke der Schneeschichten innerhalb der Schneedecke bestimmt werden.

In einer vorteilhaften Ausführungsvariante der Erfindung sind bei einer Vorrichtung mehrere Schallköpfe vorgesehen, welche vorzugsweise in Bodennähe der Vorrichtung angeordnet sind, wobei mindestens ein Schallkopf als Ultraschallsender sowie zumindest ein Schallkopf als Ultraschallempfänger ausgebildet sind.

In einer weiteren vorteilhaften Ausführungsform der erfindungsgemäßen Vorrichtung weisen die mehreren Schallköpfe jeweils nach oben gerichtete, vorzugsweise zueinander geneigte Schallabstrahlrichtungen auf, deren Abstrahlwinkel von der Normalen auf eine Grundplatte abweichen.

In dieser Ausführung mit einer Ultraschallmessung mittels einer sogenannten Mehrkopfmessung weichen die Abstrahlwinkel der Ultraschallsender/-empfänger jeweils von der Normalen auf die Grundplatte der Vorrichtung, mit der diese auf der entsprechend geneigten Geländeoberfläche am jeweiligen Aufstellort befestigt ist, ab. Somit weichen die Abstrahlwinkel weiters von der Normalen auf die Schneeschichtgrenzflächen bzw. auf die Schneeoberfläche ab und sind zueinander geneigt.

Zumindest ein Schallkopf ist dabei mit einem Ultraschallsender versehen, der in einem Abstrahlwinkel, der etwas von der Normalen auf die Schneeschichtgrenzflächen bzw. auf die Geländeoberfläche abweicht, Ultraschallwellen nach oben absendet. Die Schallwellen werden an den Schichtgrenzflächen innerhalb der Schneedecke zumindest teilweise reflektiert und treffen unter einem Reflexionswinkel, der ebenfalls von der Normalen abweicht, auf einen oder mehrere Schallköpfe, die als Ultraschallempfänger in Bodennähe angeordnet sind.

In einer Weiterbildung der Erfindung weist bei einer Vorrichtung die Schneeschichten-Messeinrichtung eine Impulsreflektometrie-Messeinrichtung umfassend zumindest eine Strom leitende Impulsreflektometrie-Messstrecke auf.

In dieser Ausführungsvariante einer stetigen Schneeschichten-Messeinrichtung wird das Messverfahren der Impulsreflektometrie angewendet, welches ein Reflexions-Messverfahren ist, das sich besonders gut zur breitbandigen Untersuchung beispielsweise von Leitungen, Leitungsabschlüssen sowie Komponenten und Netzwerken eignet. Ein elektrischer Impuls mit einer möglichst kurzen Anstiegszeit wird dabei in ein Messobjekt, beispielsweise eine Leitung mit einem unbekannten Widerstand, geleitet. Der Impuls läuft dabei den elektrischen Leiter entlang. An Störstellen entlang des Leiters, die Unstetigkeiten in der elektrischen Impedanz verursachen, wird der Impuls teilweise reflektiert bzw. bei einem Kurzschluss gänzlich ausgelöscht. Reflexionen des Impulses können von einem Impulsreflektometrie-Messgerät erfasst und ausgewertet werden.

Im Rahmen der Erfindung wird eine Impulsreflektometrie-Messeinrichtung verwendet, welche zumindest eine Strom leitende Impulsreflektometrie-Messstrecke umfasst. Die zumindest eine Impulsreflektometrie-Messstrecke ist, da sie der Witterung ausgesetzt ist und im Wesentlichen senkrecht in die Schneedecke gesteckt wird, besonders robust ausgeführt. Störstellen entlang der Impulsreflektometrie-Messstrecke, die jeweils Unstetigkeiten in der elektrischen Impedanz des Leiters verursachen, sind beispielsweise Schichtgrenzflächen zwischen benachbarten Schneeschichten.

Zweckmäßig umfasst bei einer erfindungsgemäßen Vorrichtung die Impulsreflektometrie-Messstrecke zumindest zwei elektrische Leiterbahnen, die im Wesentlichen lotrecht sowie in einem konstanten Abstand zueinander angeordnet sind.

Es ist beispielsweise denkbar, als Impulsreflektometrie-Messstrecke zwei stabile, elektrisch leitende Metallstäbe mit jeweils gleichem Querschnitt zu verwenden, welche in regelmäßigen Abständen mit Abstandhaltern verbunden sind. Die Metallstäbe, welche zwei elektrische Leiterbahnen bilden, sind somit in einem konstanten Abstand zueinander und somit parallel zueinander angeordnet. Der Gestaltung des Querschnittprofils der Leiterbahnen sind keine Grenzen gesetzt. Beispielsweise können Leiterbahnen bildende Metallstäbe mit kreisförmigen, elliptischen oder quadratischen Querschnittsflächen verwendet werden. Wichtig ist, dass die elektrischen Leiterbahnen zum Schutz vor Kurzschlussströmen an ihren Außenflächen mit einer Isolierungsschicht mit jeweils gleichmäßiger Isolierschichtdicke versehen sind. Als Isolierungsschicht dient beispielsweise eine gleichmäßige Lackschicht oder eine Schicht eines Schrumpfschlauchs aus Kunststoff, die außenseitig auf den Metallstäben aufgebracht sind.

Vorteilhaft umfasst bei einer Vorrichtung gemäß der Erfindung die Impulsreflektometrie-Messstrecke zumindest einen länglichen Trägerkörper vorzugsweise aus Kunststoff, welcher mit einer wasserdichten Außenschicht versehen ist.

In dieser Ausführungsvariante einer Impulsreflektometrie-Messstrecke wird ein Trägerkörper vorzugsweise aus Kunststoff verwendet, an dessen Außenseiten zwei voneinander gleichmäßig beabstandete elektrische Leiterbahnen angeordnet sind. Die auf dem Kunststoff angeordneten Leiterbahnen sind zum Schutz vor Kurzschlussströmen ebenfalls mit einer wasserdichten Isolierungsschicht ummantelt. Eine solche wasserdichte Außenschicht ist bei einer Impulsreflektometrie-Messstrecke, die im Rahmen der Erfindung in einer Schneedecke eingesteckt ist, unerlässlich.

Zur Herstellung des Trägerkörpers werden Materialien bevorzugt, welche eine schlechte Wärmeleitfähigkeit aufweisen. Die Impulsreflektometrie-Messstrecke bleibt während der Wintermonate permanent im Wesentlichen lotrecht in die Schneedecke eingesteckt und steht mit der umgebenden Schneedecke in direktem Kontakt. Der Trägerkörper soll sich nicht durch die Sonneneinstrahlung aufheizen, um ein Abschmelzen der umgebenden Schneedecke rund um die Impulsreflektometrie-Messstrecke zu vermeiden. Die Messung des Schneeschichtenprofils ist nur dann aussagekräftig, wenn die Impulsreflektometrie-Messstrecke bündig von der Schneedecke umgeben ist.

Somit werden Trägerkörper aus Kunststoff bevorzugt. Die Gestaltung des Querschnittprofils des Trägerkörpers kann beispielsweise kreisförmig, polygonal oder tropfenförmig ausgeführt sein. Ebenso ist es im Rahmen der Erfindung denkbar, ein innen hohles Kunststoffrohr oder ein im Inneren mit einem weiteren Material, beispielsweise mit einem Polyurethan-Schaum, verfülltes Kunststoffrohr, als Trägerkörper einzusetzen.

Um Schwingungen des nach oben über die Schneeoberfläche der Schneedecke vorragenden freien Endes der Impulsreflektometrie-Messstrecke, die beispielsweise durch starken Wind hervorgerufen werden, zu vermeiden, wird das freie obere Ende des Trägerkörpers vorteilhaft abgespannt. Dazu kann der Trägerkörper beispielsweise mittels Spannseilen am Vorrichtungsmast der erfindungsgemäßen Vorrichtung befestigt werden.

In einer Fortbildung der Erfindung ist vorgesehen, dass bei einer Vorrichtung die Schneeschichten-Messeinrichtung zumindest einen Radarsender/-empfänger aufweist.

In dieser Ausführungsvariante werden ein oder mehrere Radarsender/-empfänger zur Erfassung eines stetigen Schneeschichtenprofils eingesetzt, die vom Messprinzip ähnlich wie ein oder mehrere Ultraschallsender/-empfänger funktionieren. Anstelle von Ultraschallwellen werden von zumindest einem Radarsender Radarwellen ausgesendet, die an Unstetigkeitsstellen in der Schneedecke, beispielsweise an Schichtgrenzflächen zwischen benachbarten Schneeschichten, teilweise reflektiert werden und von einem oder mehreren Radarempfängern detektiert werden.

Ebenso ist es im Rahmen der Erfindung denkbar, sowohl Ultraschallsender/-empfänger, als auch Radarsender/-empfänger zur Erfassung eines Schneeschichtenprofils einzusetzen. Weiters kann bei einer erfindungsgemäßen Vorrichtung zusätzlich zu mindestens einer Schneeschichten-Messeinrichtung, welche einen Ultraschallsender/-empfänger und/oder einen Radarsender/-empfänger aufweist, zumindest eine Impulsreflektometrie-Messeinrichtung als Schneeschichten-Messeinrichtung vorgesehen sein.

Es ist bevorzugt, eine erfindungsgemäße Vorrichtung weiterhin umfassend zumindest eine Schneefließgeschwindigkeits-Messeinrichtung vorzusehen, mit der ein stetiges Schneefließgeschwindigkeitsprofil erfassbar ist.

Zweckmäßig umfasst bei einer Vorrichtung gemäß der Erfindung die Schneefließgeschwindigkeits-Messeinrichtung zumindest ein Fließgeschwindigkeitsmessrad, welches eine, vorzugsweise ungerade, Anzahl an radial abstehenden Fließgeschwindigkeitsmessstiften aufweist, welche seitlich aus dem Vorrichtungsmast hervorragen.

Gemäß einem weiteren Merkmal der Erfindung ist bei einer Vorrichtung jedes Fließgeschwindigkeitsmessrad mit einer Winkelgebereinrichtung versehen, welche eine Drehbewegung des Fließgeschwindigkeitsmessrads in einen elektrischen Impuls überträgt.

Vorteilhaft sind bei einer erfindungsgemäßen Vorrichtung mehrere Schneefließgeschwindigkeits-Messeinrichtungen in regelmäßigen Abständen über eine Masthöhe verteilt angeordnet.

Zweckmäßig ist eine Vorrichtung gemäß der Erfindung weiterhin mit zumindest einer Schneeanpressdruck-Messeinrichtung versehen, mit der ein auf den Vorrichtungsmast wirkender Schneedeckendruck erfassbar ist.

Vorteilhaft sind zumindest zwei oder mehrere Schneeanpressdruck-Messeinrichtungen, welche jeweils Drucksensoren umfassen, in definierten, konstanten Abständen zueinander entlang des Vorrichtungsmastes angeordnet. Somit ist ein Profilverlauf des Schneeanpressdrucks über die Höhe der Schneedecke erfassbar.

Zweckmäßig sind bei einer Vorrichtung im Rahmen der Erfindung eine Messdatenverarbeitungseinrichtung und/oder eine Messdatenübertragungseinrichtung vorgesehen.

Eine Messdatenverarbeitungseinrichtung dient insbesondere zur Erfassung und Verarbeitung sämtlicher Messdaten, die mit den unterschiedlichen Messeinrichtungen der Vorrichtung zur Schneebeschaffenheitsmessung erfasst werden.

Mit einer Messdatenübertragungseinrichtung werden die erfassten Messdaten kontinuierlich oder in bestimmten zeitlichen Intervallen beispielsweise via eines GSM-Modems mittels eines Mobilfunksystems an eine Auswertestelle bzw. Archivierungsstelle übertragen.

Um eine kompakte erfindungsgemäße Vorrichtung zu erhalten, ist in einer Fortbildung der Erfindung zumindest eine Energieversorgungseinrichtung, die durch Windkraft und/oder durch direkte Umwandlung von Lichtenergie in elektrische Energie mittels Solarzellen und/oder durch eine Brennstoffzelle mit Energie versorgbar ist, vorgesehen.

In einer Ausführungsvariante einer Energieversorgungseinrichtung, die mittels Solarzellen betrieben wird, werden beispielsweise zwei oder mehrere Photovoltaik-Paneele eingesetzt. Vorzugsweise werden die Photovoltaik-Paneele unter einem Neigungswinkel an der Vorrichtung befestigt, sodass Schnee von den Paneelen von selbst abrutschen kann. Erforderlichenfalls kann eine zusätzliche Beheizung der Paneele vorgesehen werden, um ein gesichertes Abtauen auch bei starkem Schneefall zu gewährleisten.

In einer Ausführung einer Energieversorgungseinrichtung mittels Windkraft ist beispielsweise ein Windgenerator vorgesehen, der an der Vorrichtung selbst und in deren Nähe vorgesehen wird.

Weiters ist es möglich, die erfindungsgemäße Vorrichtung in einem reinen Netzbetrieb zu betreiben oder zusätzlich eine Stromversorgung zur Versorgung durch eine der oben genannten Energieversorgungseinrichtungen vorzusehen.

Zweckmäßig ist bei einer Vorrichtung gemäß der Erfindung der Vorrichtungsmast an einer Grundplatte mit Befestigungsankern an einem Geländeboden befestigbar.

Zusätzlich zur Befestigung des Vorrichtungsmastes an einer Grundplatte mittels Befestigungsankern kann es erforderlich sein, zusätzliche Befestigungspunkte am Vorrichtungsmast vorzusehen. So kann es beispielsweise bei der Aufstellung einer erfindungsgemäßen Vorrichtung im hochalpinen, steilen Gelände erforderlich sein, den Vorrichtungsmast zusätzlich mittels Abspannseilen, die wiederum mit Befestigungsankern im Gelände befestigt sind, zu sichern. Somit ist es besonders einfach möglich, auch in einem steilen Geländeabschnitt den Vorrichtungsmast vorzugsweise jeweils normal zur Geländeoberfläche des Aufstellorts zu positionieren.

Vorteilhaft weist bei einer erfindungsgemäßen Vorrichtung der Vorrichtungsmast ein im Wesentlichen dreieckiges Querschnittsprofil auf. Vorzugsweise entspricht das Querschnittsprofil einem gleichschenkeligen Dreieck, wobei eine bergseitige Vorderkante zwischen den beiden Schenkeln des gleichschenkeligen Dreiecks angeordnet ist. Die beiden Schenkel bilden somit im Querschnitt jeweils die längeren Seitenflächenkanten des Vorrichtungsmastes.

Der Vorrichtungsmast wird dabei vorzugsweise normal zur Geländeoberfläche des jeweiligen Aufstellorts so positioniert, dass eine Längskante des Vorrichtungsmasts bergwärts ausgerichtet ist. Bergabwärts rutschender Schnee gleitet somit seitlich entlang den beiden Seitenflächen des Vorrichtungsmastes an diesem vorbei. Die auf den Vorrichtungsmast wirkende Widerstandskraft des rutschenden Schnees ist somit minimal.

In einer weiteren vorteilhaften Ausführung der Erfindung ist bei einer Vorrichtung zumindest eine Schneefeuchte-Messeinrichtung umfassend paarweise angeordnete Feuchtemessfühler, welche in einem definierten Abstand zueinander entlang der Masthöhe angeordnet sind und aus dem Vorrichtungsmast hervorragen, vorgesehen, wobei die Bestimmung der Schneefeuchte mittels Erfassung der elektrischen Kapazität zwischen den paarweise angeordneten Feuchtemessfühlern erfolgt. Bei einer solchen Schneefeuchte-Messeinrichtung, welche nach einem kapazitiven Messverfahren erfolgt, werden pro Schneefeuchte-Messeinrichtung jeweils paarweise zwei Feuchtemessfühler so montiert, dass jeweils einige Millimeter oder Zentimeter aus dem Mast in den Schnee ragen. Zwischen jeweils einem Paar dieser Feuchtemessfühler wird eine hochfrequente Spannung angelegt, welche sich je nach elektrischer Kapazität des umgebenden Mediums, beispielsweise Schnee, ändert. Anhand dieser Änderungen können die Kapazität und damit der Feuchtegehalt des zwischen den Feuchtemessfühlern liegenden Mediums bzw. der Schneedecke bestimmt werden. Die dabei gemessene elektrische Kapazität ist direkt proportional zum Wassergehalt der Schneedecke. Die Schneefeuchte-Messeinrichtungen können an beliebigen Seiten des Mastes montiert werden, wobei jeweils mindestens zwei Feuchtemessspitzen pro Messeinrichtung vorgesehen sind. Bevorzugt werden die Schneefeuchte-Messeinrichtungen jedoch an der Vorderkante des Mastes angeordnet.

In einer weiteren vorteilhaften Ausführung der Erfindung sind die Schneetemperatur-Messeinrichtungen und die Schneefeuchte-Messeinrichtungen baulich kombiniert, wobei jeweils eine der Messspitzen der Schneefeuchte-Messeinrichtungen gleichzeitig als Temperaturfühler der Schneetemperatur-Messeinrichtungen ausgeführt ist.

Zweckmäßig umfasst bei einer Vorrichtung gemäß der Erfindung die Schneefließgeschwindigkeits-Messeinrichtung zumindest eine Kameraeinrichtung, welche im Vorrichtungsmast integriert ist. Mittels einer oder mehrerer im Mast integrierten Kameraeinrichtungen, welche auf den seitlich am Mast vorbeigleitenden Schnee gerichtet sind, können beispielsweise mittels eines geeigneten Bildbearbeitungsprogramms jeweils einzelne markante Schneekristalle und/oder sonstige markante Partikel der Schneedecke erfasst und deren Bewegung entlang des Vorrichtungsmastes dokumentiert werden. Anhand einzelner fotographisch erfasster Schneekristalle oder Partikel ist somit die Schneefließgeschwindigkeit bestimmbar. Die zumindest eine Kameraeinrichtung befindet sich dazu vor Witterungseinflüssen geschützt beispielsweise hinter einer Schutzglasscheibe im Inneren des Vorrichtungsmastes.

In einer Weiterbildung der Erfindung umfasst bei einer Vorrichtung jede Kameraeinrichtung zumindest einen Kamerasensor und/oder zumindest einen Lasersensor zur Erfassung der Schneefließgeschwindigkeit. In der Ausführung mit einem Kamerasensor umfasst jede Kameravorrichtung vorteilhaft eine eigene Lichtquelle, um die vorbeifließenden Schneekristalle oder Schmutzpartikel photographisch erfassen zu können. In der Ausführung mit einem Lasersensor sendet einen Laserstrahl, welcher vorzugsweise eine Wellenlänge des nicht sichtbaren Lichtbereichs aufweist, horizontal in die Schneedecke aus. Die Reflexionen an den Schneekristallen treffen auf einen in der Nähe einer Laserdiode befindlichen lichtempfindlichen Sensor. Bei Bewegung der Schneedecke wird gleichzeitig auch der Auftreffpunkt der Reflexionen auf dem lichtempfindlichen Sensor verändert. Dadurch können die Bewegung der Schneekristalle und somit die Schneefließgeschwindigkeit berechnet werden. Im Rahmen der Erfindung ist es auch möglich, Kameraeinrichtungen zur Erfassung der Schneefließgeschwindigkeit vorzusehen, bei denen sowohl Kamerasensoren, als auch Lasersensoren vorgesehen sind.

Weitere Einzelheiten, Merkmale und Vorteile der Erfindung ergeben sich aus der nachfolgenden Erläuterung eines in den Zeichnungen schematisch dargestellten Ausführungsbeispiels. In den Zeichnungen zeigen:
- Fig. 1 in einer isometrischen Ansicht von schräg oben eine erfindungsgemäße Vorrichtung zur Schneebeschaffenheitsmessung;
- Fig. 2 in einer isometrischen Ansicht von schräg oben eine erfindungsgemäße Vorrichtung, die teilweise von einer Schneedecke bedeckt ist;
- Fig. 3 in einer isometrischen Ansicht von der Seite eine Schneefließgeschwindigkeits-Messeinrichtung als Detail einer erfindungsgemäßen Vorrichtung;
- Fig. 4 in einer isometrischen Detailansicht einer erfindungsgemäßen Vorrichtung mehrere Schneefließgeschwindigkeits-Messeinrichtungen, die jeweils außenseitig an der Vorrichtung hervorragen;
- Fig. 5 in einer mit Fig. 4 vergleichbaren Ansicht die Vorrichtung mit abgenommenen Wartungsabdeckungen;
- Fig. 6 bis 8 jeweils in Schnittansichten den Verlauf von Schallwellen während einer Messung von übereinander gelagerten Schneeschichten mittels eines Ultraschallsender/- empfängers;
- Fig. 9 in Diagrammform ein Signalbild eines Ultraschallsender/-empfängers während einer Messung von übereinander gelagerten Schneeschichten;
- Fig. 10 in einer Schnittansicht eine weitere Messanordnung einer Messung von übereinander gelagerten Schneeschichten mittels mehrerer Ultraschallsender/-empfänger;
- Fig. 11 in einem Blockschaltbild eine Anordnung zur Schichtenmessung von übereinander gelagerten Schneeschichten mittels eines Ultraschallsender/-empfängers;
- Fig. 12 in einem Blockschaltbild eine Anordnung zur Schichtenmessung von übereinander gelagerten Schneeschichten mittels mehrerer Ultraschallsender/-empfänger;
- Fig. 13 in einem Blockschaltbild eine Anordnung zur Schichtenmessung von übereinander gelagerten Schneeschichten mittels einer Impulsreflektometrie-Messeinrichtung;
   Fig. 14 in einer isometrischen Ansicht von schräg oben eine Impulsreflektometrie-Messstrecke.

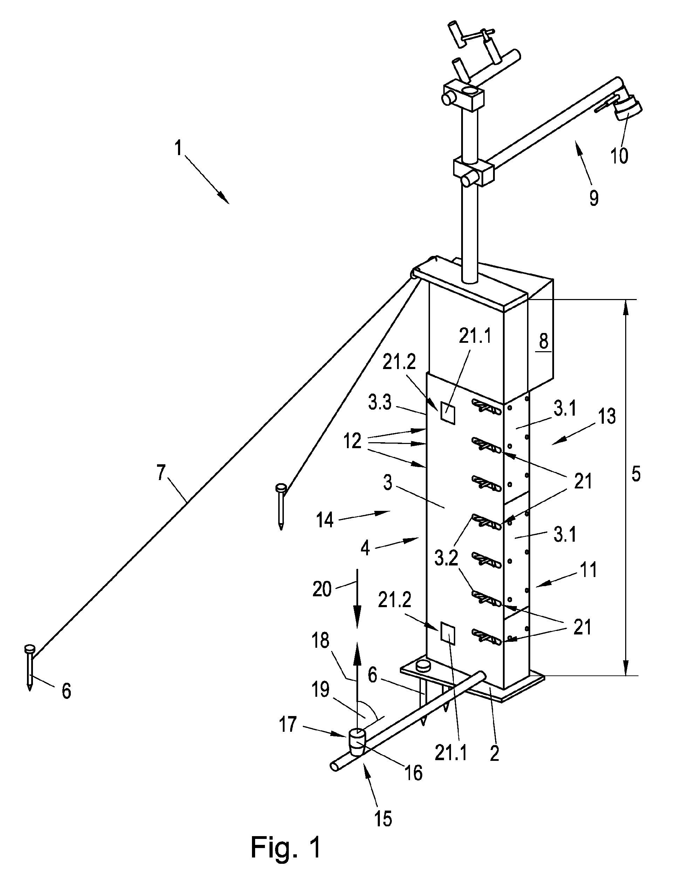

Fig. 1 zeigt eine Darstellung eines Ausführungsbeispiels einer erfindungsgemäßen Vorrichtung 1 zur Schneebeschaffenheitsmessung. Eine Grundplatte 2 der Vorrichtung 1 ist an der Unterseite eines Vorrichtungsmastes 3 befestigt. Der Vorrichtungsmast 3 ist mit mehreren Wartungsabdeckungen 3.1 sowie mit Ausnehmungen 3.2, welche an seinen Seitenflächen angeordnet sind, versehen. Der Vorrichtungsmast 3 weist ein im Wesentlichen dreieckiges Querschnittsprofil 4 in Form eines gleichschenkeligen Dreiecks auf. Um eine sichere Befestigung des länglichen Masts 3, der eine Masthöhe 5 aufweist, zu gewährleisten, sind an der Grundplatte 2 mehrere Durchgangsbohrungen zur Aufnahme von Befestigungsankern 6 vorgesehen. Die Befestigungsanker 6 werden beispielsweise in einen hier nicht dargestellten festen Untergrund gebohrt oder in diesen eingeschlagen oder mittels eines Bindemittels, beispielsweise mittels Beton, in einem festen Untergrund eingegossen. Zur Sicherung des Vorrichtungsmastes 3 sind weiters Spannseile 7 vorgesehen, wobei jeweils ein Spannseilende am oberen Ende des Vorrichtungsmastes 3 befestigt ist und das jeweils andere, freie Spannseilende mittels weiterer Befestigungsanker 6 in einem festen Untergrund befestigbar sind.

Solcherart mittels Spannseilen 7 abgespannt befestigt ist es möglich, den Vorrichtungsmast 3 auch in einem unwegsamen, beispielsweise steilen, hochalpinen, Gelände im Wesentlichen normal zur jeweiligen Geländeoberfläche des Aufstellorts zu befestigen. Je nach Anforderung ist es somit vorteilhaft möglich, einen Vorrichtungsmast 3 wahlweise entweder lotrecht oder im Wesentlichen senkrecht zur Oberfläche des Geländes zu befestigen. Der Vorrichtungsmast 3 wird dabei üblicherweise im Gelände so befestigt, dass die beiden Schenkel des dreieckigen Querschnittsprofils 4 die bergaufwärts orientierten, längeren Seitenflächenkanten bilden und der Vorrichtungsmast 3 vergleichbar mit einer Pfeilform somit mit seiner zwischen den beiden Dreiecksschenkeln gelegenen Vorderkante 3.3 bergaufwärts orientiert ist. Somit bietet die Vorderkante 3.3 des Vorrichtungsmastes 3 vorteilhaft eine minimale Angriffsfläche für eine bergabwärts rutschende Schneedecke.

Am oberen Ende des Vorrichtungsmastes 3, welches üblicherweise aus einer hier nicht dargestellten Schneedecke über eine Schneeoberfläche hervorragt, ist hier eine Einhausung 8 vorgesehen. Die Einhausung 8 dient zur Aufnahme von Messinstrumenten, die Daten von meteorologischen Messeinrichtungen 9, wie beispielsweise die Windgeschwindigkeit, Windrichtung, Lufttemperatur, Luftdruck, Luftfeuchte, Globalstrahlung, Schneedeckenabstrahlung, Schneeoberflächentemperatur, die Schneehöhe oder die Niederschlagsmenge erfassen. Die meteorologischen Messeinrichtungen 9 sowie eine Schneehöhen-Messeinrichtung 10 sind an Halterungen an der Oberseite des Vorrichtungsmastes 3 befestigt und ragen somit jedenfalls über die Schneedecke hervor.

In einem unteren Mastabschnitt ist eine Schneewiegeeinrichtung 11 vorgesehen. Mehrere Schneetemperatur-Messeinrichtungen 12 sind entlang der Vorderkante 3.3 des Vorrichtungsmastes 3 angeordnet und ermöglichen es, einen Temperaturverlauf über das Schneehöhenprofil aufzunehmen. Ebenso ist in dieser in Fig. 1 gezeigten Ausführungsform eine Schneefeuchte-Messeinrichtung 14 vorgesehen. Die erfindungsgemäße Vorrichtung 1 ist weiters mit einer Schneeschichten-Messeinrichtung 15 zur Erfassung eines stetigen Schneeschichtenprofils ausgestattet. Dazu ist an einer Halterung in Bodennähe des Vorrichtungsmastes 3 ein Schallkopf 16 mit einem Ultraschallsender/-empfänger 17 vorgesehen. Der Ultraschallsender 17 ist so positioniert, dass Ultraschallsignale in einer Schallabstrahlrichtung 18 im Wesentlichen normal zur Grundplatte 2 und somit normal zur jeweiligen Geländeoberfläche nach oben ausgesendet werden. Die Ultraschallsignale treffen somit normal von unten auf Schneeschichtgrenzflächen einer Schneedecke. Ein Schallabstrahlwinkel 19, der zwischen der Schallabstrahlrichtung 18 und der Ebene der Grundplatte 2 gemessen wird, beträgt im Wesentlichen 90°.

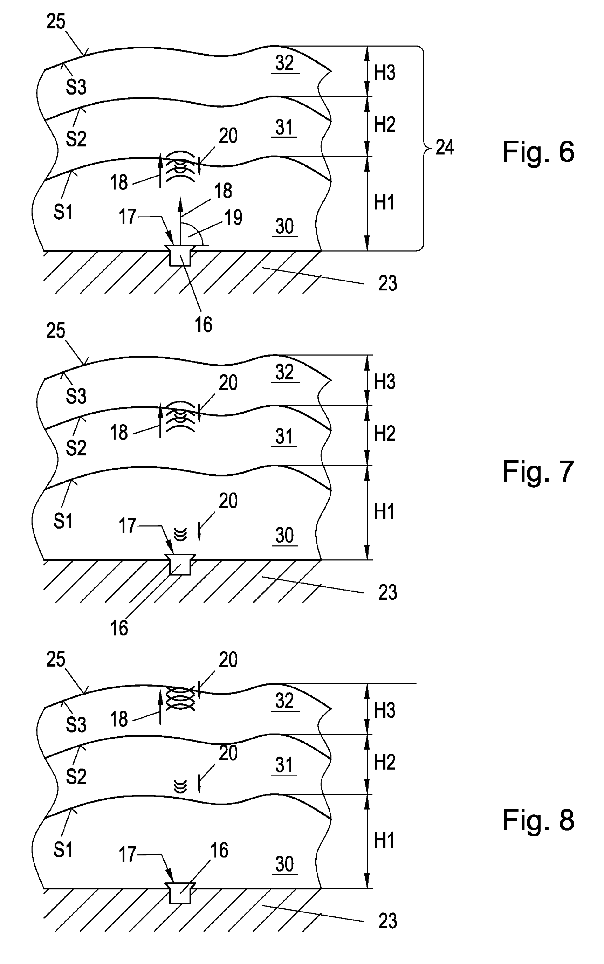

Der Ultraschallempfänger 17 dient weiters zur Erfassung von reflektierten Ultraschallsignalen, die in einer Schallreflexionsrichtung 20 reflektiert und wieder zum Ultraschallempfänger 17 gelenkt werden. Das Funktionsprinzip einer stetigen Schneeschichtenprofilmessung mittels Ultraschall wird in den folgenden Abbildungen Fig. 6 bis Fig. 10 veranschaulicht.

Im Inneren des Vorrichtungsmastes 3 sind in gleichmäßigen Abständen Schneefließgeschwindigkeits-Messeinrichtungen 21 eingebaut, die zur stetigen Aufnahme eines Schneefließgeschwindigkeitsprofils dienen. Die Funktionsweise der Schneefließgeschwindigkeits-Messeinrichtung 21, welche hier jeweils auch ein Fließgeschwindigkeitsmessrad umfasst, wird nachfolgend im Detail beschrieben.

Weiters sind in Fig. 1 Kameraeinrichtungen 21.1 dargestellt, welche zum Schutz im Inneren des Vorrichtungsmasts 3 integriert und nach außen beispielsweise durch eine Sicherheitsglasscheibe vor Witterungseinflüssen geschützt sind. Dabei werden von Kamerasensoren 21.2 einzelne markante Schneekristalle oder Partikel erfasst und deren Fließbewegung entlang des Vorrichtungsmasts 3 während einer definierten Zeitspanne aufgezeichnet. Solcherart kann ebenfalls das Schneefließgeschwindigkeitsprofil ermittelt werden.

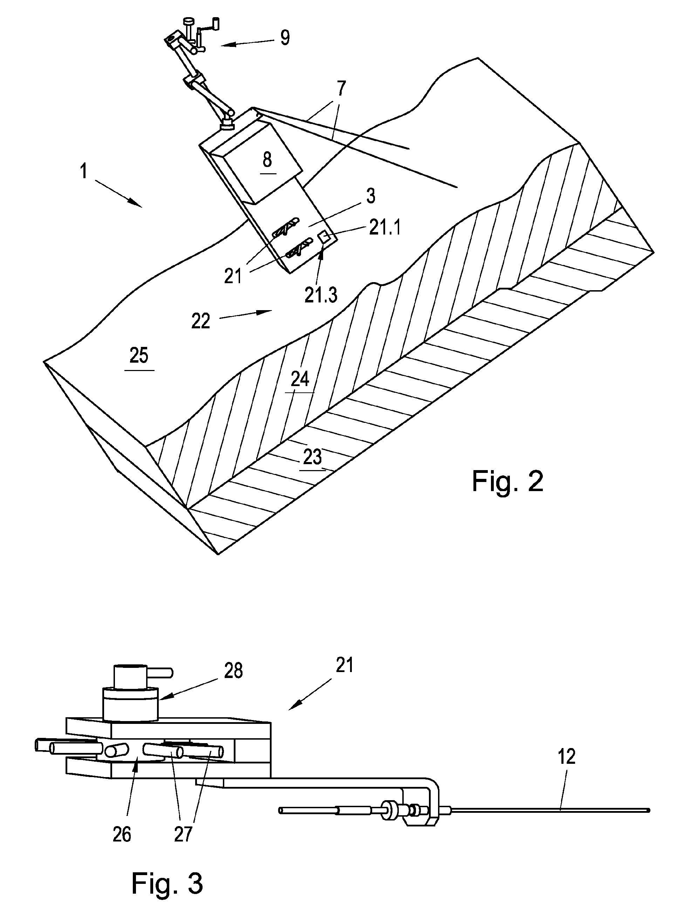

Fig. 2 veranschaulicht eine weitere Ausführungsform einer erfindungsgemäßen Schneebeschaffenheitsmessvorrichtung 1, welche anstelle eines Schallkopfs mit einem Ultraschallsender/-empfänger mit einem Radarsender/-empfänger 22 ausgestattet ist. Auch dieser Radarsender/-empfänger 22, der ähnlich einem Ultraschallsender/-empfänger wirkt und Radarwellen aussendet, ist in Bodennähe des Vorrichtungsmastes 3 angeordnet und sendet Radarwellen im Wesentlichen senkrecht zur Geländeoberfläche eines Geländebodens 23 und somit senkrecht zu einzelnen Schneeschichten einer Schneedecke 24 aus. Die Vorrichtung 1 ist in Fig. 2 am festen Geländeboden 23 beispielsweise eines geneigten Berghangs ortsfest befestigt und hier im Wesentlichen senkrecht zur Geländeoberfläche befestigt. Die Schneedecke 24 mit einer Schneeoberfläche 25, die in einer Schnittansicht dargestellt ist, bedeckt dabei den Unterteil des Vorrichtungsmastes 3. Ebenso ist es im Rahmen der Erfindung möglich, den Vorrichtungsmast 3 lotrecht im Gelände zu befestigen.

Weiters ist in Fig. 2 eine Kameraeinrichtung 21.1 zur Erfassung des Schneefließgeschwindigkeitsprofils dargestellt, welche zum Schutz im Inneren des Vorrichtungsmasts 3 integriert und nach außen durch eine Sicherheitsglasscheibe vor Witterungseinflüssen geschützt ist. Es werden hier von Lasersensoren 21.3 einzelne markante Schneekristalle oder Partikel erfasst und deren Fließbewegung entlang des Vorrichtungsmasts 3 während einer definierten Zeitspanne aufgezeichnet. Weitere Kameraeinrichtungen 21.1 sind unterhalb der Schneeoberfläche 25 in definierten Abständen entlang des Vorrichtungsmasts 3 angeordnet.

Fig. 3 zeigt in einer isometrischen Ansicht von der Seite eine Schneefließgeschwindigkeits-Messeinrichtung 21 als Detail einer erfindungsgemäßen Vorrichtung 1. Derartige Schneefließgeschwindigkeits-Messeinrichtungen 21 sind im Inneren des Vorrichtungsmastes 3 in gleichmäßigen Abständen eingebaut und dienen zur stetigen Aufnahme eines Schneefließgeschwindigkeitsprofils. Jede Schneefließgeschwindigkeits-Messeinrichtung 21 umfasst zumindest ein Fließgeschwindigkeitsmessrad 26, welches eine ungerade Anzahl an radial abstehenden Fließgeschwindigkeitsmessstiften 27 aufweist.

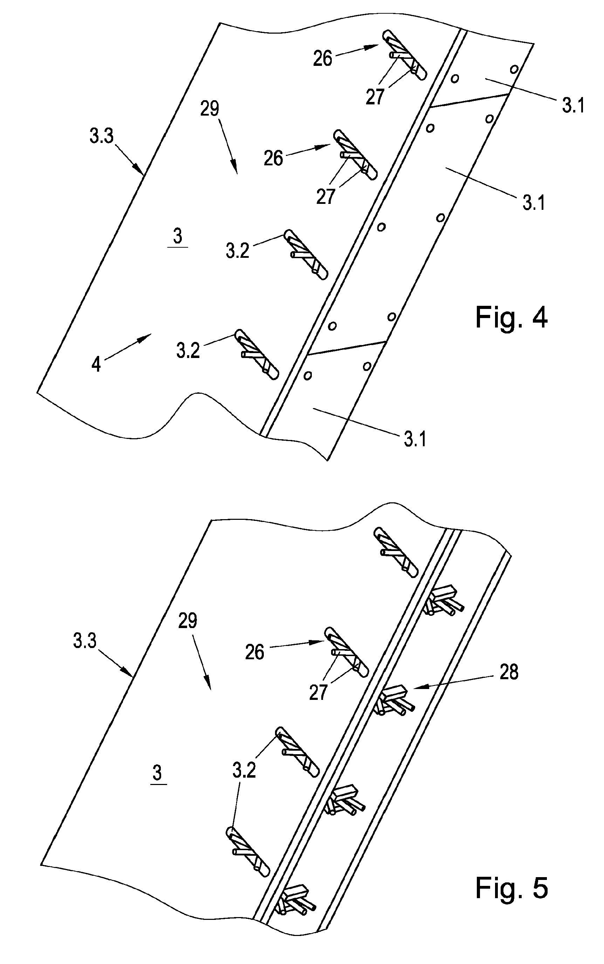

Wie in den Abbildungen Fig. 4 und Fig. 5 veranschaulicht wird, sind die Fließgeschwindigkeitsmessstifte 27 so dimensioniert, dass sie durch die seitlichen Ausnehmungen 3.2 des Vorrichtungsmastes hervorragen. Durch seitlich an den beiden Seitenflanken des Vorrichtungsmastes 3 entlang gleitenden Schnee werden die Fließgeschwindigkeitsmessräder 26 bewegt bzw. in Drehung versetzt. Jedes Fließgeschwindigkeitsmessrad 26 ist dazu mit einer Winkelgebereinrichtung 28 versehen, welche eine Drehbewegung des Fließgeschwindigkeitsmessrads 26 in einen elektrischen Impuls überträgt. Jede Schneefließgeschwindigkeits-Messeinrichtung 21 ist hier weiters mit einer Schneetemperatur-Messeinrichtung 12 versehen, welche einen Temperaturfühler umfasst, der so im Inneren des Vorrichtungsmastes 3 angeordnet ist, dass die Spitze des Temperaturfühlers an der Vorderkante 3.3 des Mastes nach außen in die Schneedecke ragt. Somit ragt bei jeder Schneefließgeschwindigkeits-Messeinrichtung 21 jeweils ein Temperaturfühler der Schneetemperatur-Messeinrichtung 12 an der Vorderkante 3.3 des Vorrichtungsmastes 3 nach außen in die Schneedecke. Weiters ist es im Rahmen der Erfindung möglich, die Temperaturfühler auch an den Seitenflächen des Vorrichtungsmastes anzubringen. Aufgrund der voneinander in definierten Abständen positionierten Temperaturfühler ist es somit möglich, ein sehr genaues Temperaturprofil über die Höhe der Schneedecke 24 aufzunehmen.

In Fig. 5 wird der Vorrichtungsmast 3 im Unterschied zu Fig. 4 mit abgenommenen Wartungsabdeckungen 3.1 gezeigt. Die Wartungsabdeckungen 3.1 sind an der der Vorderkante 3.3 gegenüberliegenden Seite des Vorrichtungsmastes 3, welche der kürzeren Seitenflächenkante des dreieckförmigen Querschnittsprofils 4 entspricht, angeordnet.

Die Abbildungen Fig. 6 bis Fig. 8 dienen zur Veranschaulichung des Messprinzips einer stetigen Schneeschichtenprofilmessung mittels eines Ultraschallsender/-empfängers 17. Der Schallkopf 16 des Ultraschallsender/-empfängers 17 ist bodennah angeordnet, wobei die Schallabstrahlrichtung 18 im Wesentlichen normal zur hier waagrecht skizzierten Geländeoberfläche des Geländebodens 23 nach oben ausgerichtet ist. Der Schallabstrahlwinkel 19 steht jeweils normal zur Geländeoberfläche bzw. normal zur Ebene der Grundplatte 2. Ein Ultraschallsignal, welches in Schallabstrahlrichtung 18 nach oben ausgestrahlt wird, wird an einer ersten Schneeschichtgrenze S 1 zwischen einer unteren Schneeschicht 30 und einer darüberliegenden, mittleren Schneeschicht 31 teilweise reflektiert und gelangt in Schallreflexionsrichtung 20 wieder zurück zum Ultraschallsender/- empfänger 17.

In Fig. 7 ist ein in Schallabstrahlrichtung 18 ausgesandtes Ultraschallsignal schematisch dargestellt, wie dieses innerhalb der Schneedecke 24 auf eine weitere, zweite Schneeschichtgrenze S2 trifft, die zwischen einer mittleren Schneeschicht 31 und einer oberen Schneeschicht 32 auftritt. An dieser Schneeschichtgrenze S2 zwischen der mittleren Schneeschicht 31 und der oberen Schneeschicht 32 wird das Ultraschallsignal wiederum aufgrund von Dichteunterschieden zwischen den beiden Schneeschichten 31 und 32 teilweise reflektiert und trifft in Schallreflexionsrichtung 20 wieder auf den Ultraschallsender/-empfänger 17.

Ebenso verhält es sich gemäß Fig. 8 mit dem Ultraschallsignal, welches an der Schneeschichtgrenze S3 zwischen der oberen Schneeschicht 32 und der Schneeoberfläche 25, also an der Grenzfläche zwischen der Schneeoberfläche 25 und einer darüber befindlichen Umgebungsluft in Schallreflexionsrichtung 20 total reflektiert wird und wieder zurück zum Ultraschallsender/-empfänger 17 gelangt.

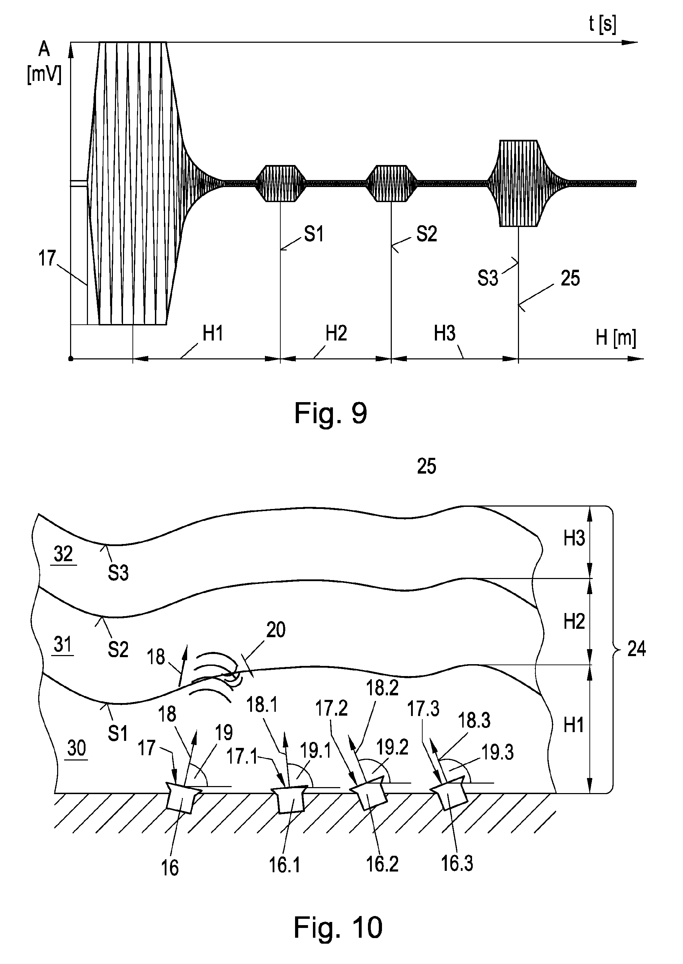

Fig. 9 zeigt in Diagrammform ein Signalbild eines Ultraschallsender/-empfängers 17 während einer Messung von übereinander gelagerten Schneeschichten 30, 31, 32. Auf einer Abszissenachse ist eine Zeitdauer t beispielsweise mit der Einheit s aufgetragen, die das vom Ultraschallsender/-empfänger 17 ausgesandte Schallsignal benötigt, um an einer Schneeschichtgrenze S1, S2 bzw. S3 reflektiert und wiederum vom Ultraschallsender/- empfänger 17 empfangen zu werden. Auf einer Ordinatenachse ist jeweils eine Signalamplitude A beispielsweise mit der Einheit mV aufgetragen. Die Signalamplituden A als Funktion der Zeit t sind in eine Schneehöhe H - beispielsweise mit der Einheit m - der Schneedecke 24 bzw. in die Schichthöhen H1, H2, H3 der Schneeschichten 30, 31, 32 umrechenbar.

Fig. 10 zeigt ein Funktionsprinzip der stetigen Schneeschichtenprofilmessung für eine weitere Ausführungsvariante einer erfindungsgemäßen Vorrichtung 1. Hier sind mehrere Schallköpfe 16, 16.1, 16.2, 16.3 in Bodennähe der Vorrichtung 1 angeordnet. Mindestens ein Schallkopf 16 umfasst dabei einen Ultraschallsender 17. Sämtliche Schallköpfe 16, 16.1, 16.2, 16.3 sind als Ultraschallempfänger 17, 17.1, 17.2, 17.3 ausgebildet. Die Schallköpfe 16, 16.1, 16.2, 16.3 sind dabei so positioniert, dass sie jeweils nach oben gerichtete sowie zueinander geneigte Schallabstrahlrichtungen 18, 18.1, 18.2, 18.3 aufweisen. Die Abstrahlwinkel 19, 19.1, 19.2, 19.3 der Schallabstrahlrichtungen 18, 18.1, 18.2, 18.3 weichen dabei jeweils von der Normalen auf die Geländeoberfläche bzw. von der Normalen auf die Schneeschichten 30, 31, 32 ab. Ein vom Ultraschallsender 17 in Schallabstrahlrichtung 18 schräg nach oben ausgesandtes Ultraschallsignal wird an einer ersten Schneeschichtgrenze S 1 zwischen der unteren Schneeschicht 30 und einer darüber liegenden Schneeschicht 31 in Pfeilrichtung 20 reflektiert und von den Ultraschallempfängern 17, 17.1, 17.2, 17.3 empfangen.

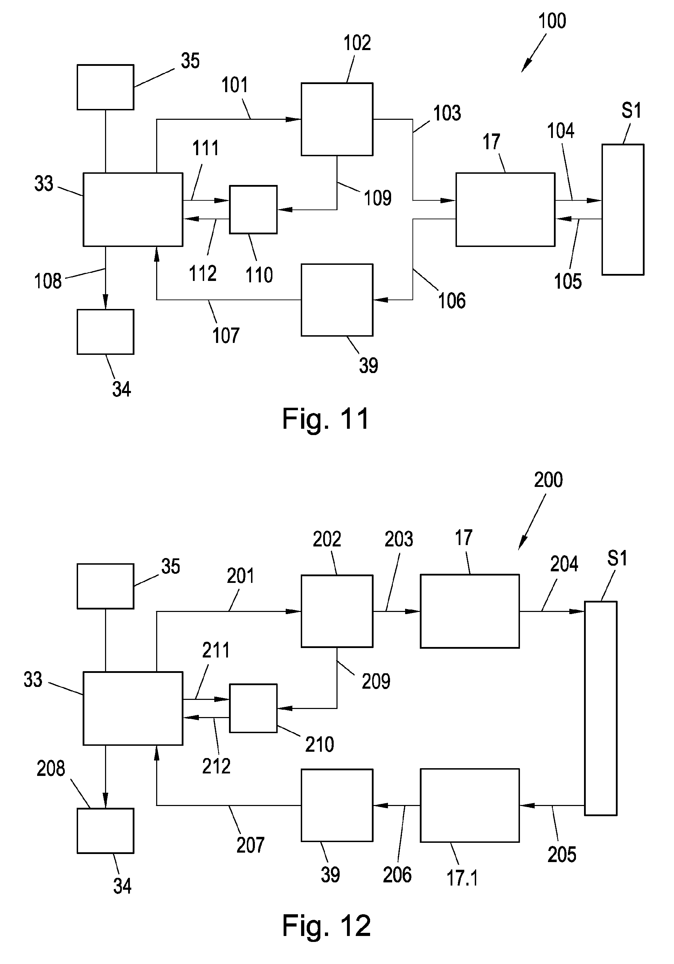

Fig. 11 zeigt in einem Blockschaltbild eine Anordnung 100 zur Schichtenmessung von übereinander gelagerten Schneeschichten mittels eines Ultraschallsender/-empfängers.

Von einer Messdatenverarbeitungseinrichtung 33 wird ein Startsignal 101 an einen Signal-Generator 102 gesendet. Der Signal-Generator 102 sendet daraufhin ein Ansteuersignal 103 an einen Ultraschallsender/-empfänger 17, dessen Senderteil ein Schallwellensignal 104 innerhalb einer Schneedecke aussendet. Das Schallwellensignal 104 wird an einer Schneeschichtgrenzfläche S1 teilweise reflektiert und als Reflexionssignal 105 wiederum vom Empfängerteil des Ultraschallsender/-empfängers 17 empfangen. Vom Ultraschallsender/-empfänger 17 wird das empfangene Signal daraufhin als elektrisches Schallsignal 106 an einen Analog/Digital-Wandler 39 geleitet, von welchem das digitalisierte Schallsignal 107 wieder zurück an die Messdatenverarbeitungseinrichtung 33 zur weiteren Verarbeitung gelangt. Von der Messdatenverarbeitungseinrichtung 33 werden Datenübertragungssignale 108 an eine Messdatenübertragungseinrichtung 34 geleitet und von dieser beispielsweise via Funk weiter an eine Auswertestelle übertragen. Weiters ist eine Energieversorgungseinrichtung 35, die beispielsweise mit Solarpaneelen ausgestattet ist, zum Betrieb der Messdatenerfassung und -verarbeitung vorgesehen. Vom Signal-Generator 102 wird zur Zeiterfassung ein Timer-Signal 109 an einen Timer 110 gesendet, wobei von diesem wahlweise ein Auslesesignal 112 an die Messdatenverarbeitungseinrichtung 33 gelangt oder umgekehrt ein Rücksetzsignal 111 von der Messdatenverarbeitungseinrichtung 33 an den Timer 110 ergeht.

Fig. 12 zeigt in einem Blockschaltbild eine Anordnung 200 zur Schichtenmessung von übereinander gelagerten Schneeschichten mit einem Mehrkopfsystem umfassend mehrere Ultraschallsender/-empfänger, wobei ein Ultraschallsender/-empfänger 17 als Senderteil betrieben wird, von dem ein Schallwellensignal 204 innerhalb der Schneedecke ausgesendet und an einer Schneeschichtgrenzfläche S 1 teilweise reflektiert wird. Das Reflexionssignal 205 wird von einem weiteren Ultraschallsender/-empfänger 17.1, welcher als Empfängerteil betrieben wird, empfangen und analog zum zuvor in Fig. 11 beschriebenen Ablauf weiter verarbeitet.

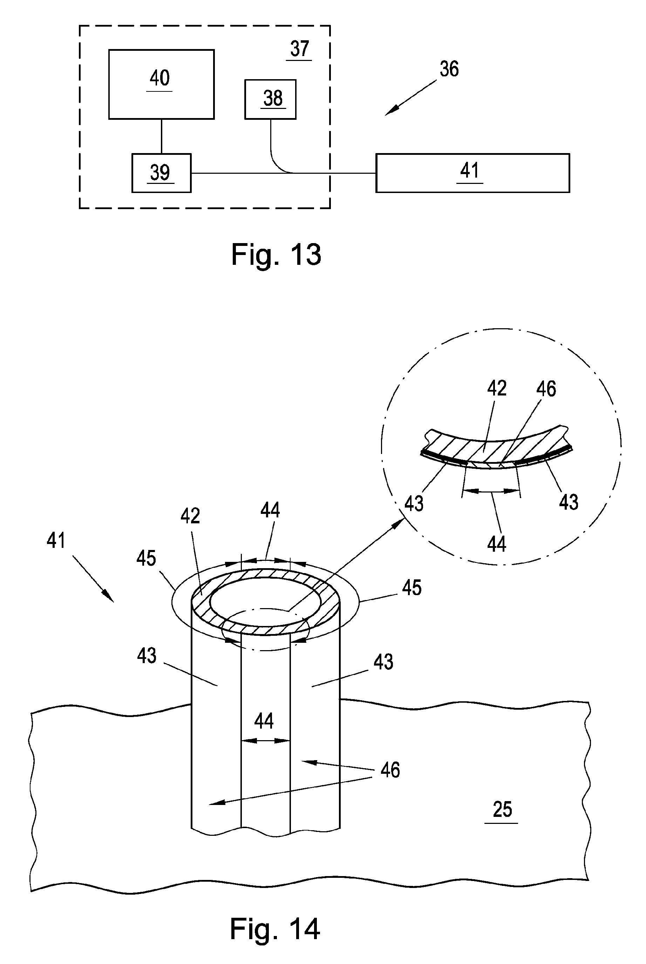

Fig. 13 zeigt in einer schematischen Darstellung als Blockschaltbild eine Impulsreflektometrie-Messeinrichtung 36. Die strichlierte Linie symbolisiert ein Impulsreflektometrie-Messgerät 37, welches als wesentliche Komponenten einen Pulsgenerator 38, einen Analog/Digital-Wandler 39 sowie eine Signalanzeigeeinrichtung 40, beispielsweise ein Oszilloskop, umfasst. Das Impulsreflektometrie-Messgerät 37 ist mit einer Impulsreflektometrie-Messstrecke 41 leitend verbunden.

Fig. 14 betrifft eine erfindungsgemäße Ausführungsform einer Impulsreflektometrie-Messstrecke 41. Diese umfasst einen Trägerkörper 42, der hier als hohles Kunststoffrohr ausgeführt ist. Somit wird mit dem Trägerkörper 42 ein möglichst geringer Wärmetransport erzielt und der Trägerkörper 42, welcher im Wesentlichen lotrecht in eine Schneedecke 25 gesteckt wird, führt nicht zum Abtauen des ihn umgebenden Schnees. An der Außenseite des länglichen Trägerkörpers 42 sind zwei elektrische Leiterbahnen 43 in einem konstanten Abstand 44 parallel zueinander angeordnet. Die Breite 45 der beiden Leiterbahnen 43 ist jeweils gleich groß. Die Leiterbahnen 43 sowie der Trägerkörper 42 sind mit einer dünnen, wasserdichten sowie isolierenden Außenschicht 46 vor Nässeeinwirkung geschützt. Durch die Außenschicht 46 werden auch Kurzschlussströme zwischen den beiden benachbarten Leiterbahnen 43 verhindert. Als Außenschicht 46 wird hier beispielsweise ein Schrumpfschlauch verwendet. Ebenso ist es denkbar, als Außenschicht 46 beispielsweise eine Lackierungsschicht oder eine Kunststoffbeschichtung vorzusehen.

Weiters ist es denkbar, andere, in den Abbildungen nicht explizit gezeigte Ausführungen einer Impulsreflektometrie-Messstrecke 41 zu verwenden. Beispielsweise ist es im Rahmen der Erfindung möglich, zwei Metallstäbe als Leiterbahnen mit Distanzhaltern in einem konstanten Abstand zueinander zu verbinden und diese als Impulsreflektometrie-Messstrecke 41 einzusetzen.

### Verzeichnis der Positionsnummern:

- 1: Schneebeschaffenheitsmessvorrichtung
- 2: Grundplatte der Vorrichtung
- 3: Vorrichtungsmast
- 3.1: Wartungsabdeckung des Vorrichtungsmastes
- 3.2: Ausnehmung des Vorrichtungsmastes
- 3.3: Vorderkante des Vorrichtungsmastes
- 4: Querschnittsprofil des Vorrichtungsmastes (dreieckig)
- 5: Höhe des Mastes
- 6: Befestigungsanker
- 7: Spannseil
- 8: Einhausung
- 9: Meteorologische Messeinrichtung
- 10: Schneehöhen-Messeinrichtung
- 11: Schneewiegeeinrichtung
- 12: Schneetemperatur-Messeinrichtung
- 13: Schneedichte-Messeinrichtung
- 14: Schneefeuchte-Messeinrichtung
- 15: Schneeschichten-Messeinrichtung
- 16: Schallkopf
- 17: Ultraschallsender/-empfänger
- 17.1: Ultraschallempfänger (bzw. 17.2; 17.3)
- 18: Schallabstrahlrichtung (in Pfeilrichtung; bzw. 18.1; 18.2; 18.3)
- 19: Schallabstrahlwinkel (bzw. 19.1; 19.2; 19.3)
- 20: Schallreflexionsrichtung (in Pfeilrichtung; bzw. 20.1; 20.2; 20.3)
- 21: Schneefließgeschwindigkeits-Messeinrichtung
- 21.1: Kameraeinrichtung
- 21.2: Bildsensor
- 21.3: Lasersensor
- 22: Radarsender/-empfänger
- 23: Geländeboden
- 24: Schneedecke
- 25: Schneeoberfläche
- 26: Fließgeschwindigkeitsmessrad
- 27: Fließgeschwindigkeitsmessstift
- 28: Winkelgebereinrichtung
- 29: Schneeanpressdruck-Messeinrichtung
- 30: Schneeschicht (bzw. 31; 32)
- 33: Messdatenverarbeitungseinrichtung
- 34: Messdatenübertragungseinrichtung
- 35: Energieversorgungseinrichtung
- 36: Impulsreflektometrie-Messeinrichtung
- 37: Impulsreflektometrie-Messgerät
- 38: Pulsgenerator
- 39: Analog/Digital-Wandler
- 40: Signalanzeigeeinrichtung (Oszilloskop)
- 41: Impulsreflektometrie-Messstrecke
- 42: Trägerkörper
- 43: Leiterbahn
- 44: Abstand zwischen Leiterbahnen
- 45: Breite einer Leiterbahn
- 46: Außenschicht
- 100: Blockfließbild Ultraschall mit einem Schallkopf
- 200: Blockfließbild Ultraschall mit mehreren Schallköpfen
- 101: Start-Signal (bzw. 201)
- 102: Signal-Generator (bzw. 202)
- 103: Ansteuersignal (bzw. 203)
- 104: Schallwellensignal (bzw. 204)
- 105: Reflexionssignal (bzw. 205)
- 106: elektrisches Schallsignal (bzw. 206)
- 107: digitalisiertes Schallsignal (bzw. 207)
- 108: Datenübertragungssignal (bzw. 208)
- 109: Timersignal (bzw. 209)
- 110: Timer (bzw. 210)
- 111: Rücksetzsignal (bzw. 211)
- 112: Auslesesignal (bzw. 212)
- A: Amplitude (in mV)
- H: Schneehöhe (in m)
- H1: Schneehöhe der Schneeschicht (in m) (bzw. H2; H3)
- S1: Schneeschichtgrenze (bzw. S2; S3)
- t: Zeit (in s)

## Patentansprüche

1. Vorrichtung (1) zur stationären Schneebeschaffenheitsmessung, umfassend zumindest eine meteorologische Messeinrichtung (9) und/oder eine Schneehöhen-Messeinrichtung (10) und/oder eine Schneewiegeeinrichtung (11) und/oder eine Schneetemperatur-Messeinrichtung (12) und/oder eine Schneedichte-Messeinrichtung (13) und/oder eine Schneefeuchte-Messeinrichtung (14) und eine Schneeschichten-Messeinrichtung (15), mit der ein stetiges Schneeschichtenprofil erfassbar ist, **dadurch gekennzeichnet, dass** die Schneeschichten-Messeinrichtung (15) eine Impulsreflektometrie-Messeinrichtung (36) umfassend zumindest eine Strom leitende Impulsreflektometrie-Messstrecke (41) aufweist.

2. Vorrichtung (1) nach Anspruch 1, **dadurch gekennzeichnet, dass** die Impulsreflektometrie-Messstrecke (41) zumindest zwei elektrische Leiterbahnen (43) umfasst, die im Wesentlichen lotrecht sowie in einem konstanten Abstand (44) zueinander angeordnet sind.

3. Vorrichtung (1) nach Anspruch 1 oder 2, **dadurch gekennzeichnet, dass** die Impulsreflektometrie-Messstrecke (41) zumindest einen länglichen Trägerkörper (42) vorzugsweise aus Kunststoff umfasst, welcher mit einer wasserdichten Außenschicht (46) versehen ist.

4. Vorrichtung (1) nach einem der Ansprüche 1 bis 3, **dadurch gekennzeichnet, dass** der Vorrichtungsmast (3) an einer Grundplatte (2) mit Befestigungsankern (6) an einem Geländeboden (23) befestigbar ist.

5. Vorrichtung (1) nach einem der Ansprüche 1 bis 4, weiterhin umfassend zumindest eine Schneefließgeschwindigkeits-Messeinrichtung (21), mit der ein stetiges Schneefließgeschwindigkeitsprofil erfassbar ist.

6. Vorrichtung (1) nach Anspruch 5, **dadurch gekennzeichnet, dass** die Schneefließgeschwindigkeits-Messeinrichtung (21) zumindest ein Fließgeschwindigkeitsmessrad (26) umfassend eine, vorzugsweise ungerade, Anzahl an radial abstehenden Fließgeschwindigkeitsmessstiften (27) aufweist, welche seitlich aus dem Vorrichtungsmast (3) hervorragen.

7. Vorrichtung (1) nach Anspruch 5 oder 6, **dadurch gekennzeichnet, dass** jedes Fließgeschwindigkeitsmessrad (26) mit einer Winkelgebereinrichtung (28) versehen ist, welche eine Drehbewegung des Fließgeschwindigkeitsmessrads (26) in einen elektrischen Impuls überträgt.

8. Vorrichtung (1) nach einem der Ansprüche 5 bis 7, **dadurch gekennzeichnet, dass** mehrere Schneefließgeschwindigkeits-Messeinrichtungen (21) in regelmäßigen Abständen über eine Masthöhe (5) verteilt angeordnet sind.

9. Vorrichtung (1) nach einem der Ansprüche 1 bis 8, weiterhin umfassend zumindest eine Schneeanpressdruck-Messeinrichtung (29), mit der ein auf den Mast (3) wirkender Schneedeckendruck erfassbar ist.

10. Vorrichtung (1) nach einem der Ansprüche 1 bis 9, **dadurch gekennzeichnet, dass** eine Messdatenverarbeitungseinrichtung (33) und/oder eine Messdatenübertragungseinrichtung (34) vorgesehen sind.

11. Vorrichtung (1) nach einem der Ansprüche 1 bis 10, **dadurch gekennzeichnet, dass** zumindest eine Energieversorgungseinrichtung (35), die durch Windkraft und/oder durch direkte Umwandlung von Lichtenergie in elektrische Energie mittels Solarzellen und/oder durch eine Brennstoffzelle mit Energie versorgbar ist, vorgesehen ist.

12. Vorrichtung (1) nach einem der Ansprüche 1 bis 11, **dadurch gekennzeichnet, dass** der Vorrichtungsmast (3) ein im Wesentlichen dreieckiges Querschnittsprofil, vorzugsweise ein Querschnittsprofil eines gleichschenkeligen Dreiecks, wobei eine bergseitige Vorderkante (3.3) zwischen den beiden Schenkeln des gleichschenkeligen Dreiecks angeordnet ist, aufweist.

13. Vorrichtung (1) nach einem der Ansprüche 1 bis 12, **dadurch gekennzeichnet, dass** zumindest eine Schneefeuchte-Messeinrichtung (14) paarweise angeordnete Feuchtemessfühler umfasst, welche in einem definierten Abstand zueinander entlang der Masthöhe (5) angeordnet sind und aus dem Vorrichtungsmast (3) hervorragen, wobei die Bestimmung der Schneefeuchte mittels Erfassung der elektrischen Kapazität zwischen den paarweise angeordneten Feuchtemessfühlern erfolgt.

14. Vorrichtung (1) nach einem der Ansprüche 5 bis 13, **dadurch gekennzeichnet, dass** die Schneefließgeschwindigkeits-Messeinrichtung (21) zumindest eine Kameraeinrichtung (21.1) umfasst, welche im Vorrichtungsmast (3) integriert ist.

15. Vorrichtung (1) nach Anspruch 14, **dadurch gekennzeichnet, dass** jede Kameraeinrichtung (21.1) zumindest einen Kamerasensor (21.2) und/oder zumindest einen Lasersensor (21.3) zur Erfassung der Schneefließgeschwindigkeit umfasst.
